# EUROPEAN PATENT SPECIFICATION

(11) **EP 1 753 497 B1**
(45) Date of publication and mention of the grant of the patent: **30.07.2008**
(21) Application number: 05751918.3
(22) Date of filing: 20.05.2005
(51) Int. Cl.: A61M 25/10, A61M 25/00, A61F 2/06

(54) **FOLDED BALLOON FOR CATHETER**
GEFALTETER BALLON FÜR EINEN KATHETER
BALLON PLIÉ POUR CATHÉTER

(30) Priority: 21.05.2004 US 572757 P
(43) Date of publication of application: 21.02.2007
(73) Proprietor: Medtronic Vascular, Inc., Santa Rosa, CA 95403 (US)
(72) Inventor: GOSHGARIAN, Justin, Santa Rosa, CA 95405 (US); MILLER, Aaron, San Francisco, CA 94112 (US)
(74) Representative: Hughes, Andrea Michelle
(86) International application number: PCT/US2005/017896
(87) International publication number: WO 2005/113058

(56) References cited:
- US-A- 6 013 092
- US-A1- 2003 083 579

## Description

### Background of the Invention

### Field of the Invention

The present invention is related to balloons, and more particularly, to folded balloons for use with low profile balloon catheter systems.

### Background Art

Balloon catheters are used to treat coronary disease. In one common application, a balloon catheter is used in a percutaneous transluminal coronary angioplasty (PTCA) procedure. In a PTCA procedure, the balloon catheter is threaded through an artery to a site of a lesion. The balloon is inflated to compress plaque associated with coronary artery disease against the artery's walls. This creates a larger opening in the artery and helps restore adequate blood flow. The balloon is then deflated and withdrawn from the vessel.

Balloon catheters are also used in combination with stents to treat coronary disease. A stent is a miniature mesh tube, typically made from steel. The balloon catheter and stent are threaded through an artery to the site of a lesion. The balloon is inflated to expand the stent. The stent is flexible, yet strong enough to remain in place after the balloon is deflated and cleared. Once the stent is in place, the balloon is deflated and withdrawn from the vessel.

Upon deflation, the balloon tends to re-form to a folded, wrapped state due to its shape memory. In some cases, pressures in the vessel can cause the number of folds that re-form upon deflation to be different from the number of initial folds in the balloon. Larger diameter balloons with higher numbers of folds are especially more likely to re-form to a lesser number of folds upon deflation.

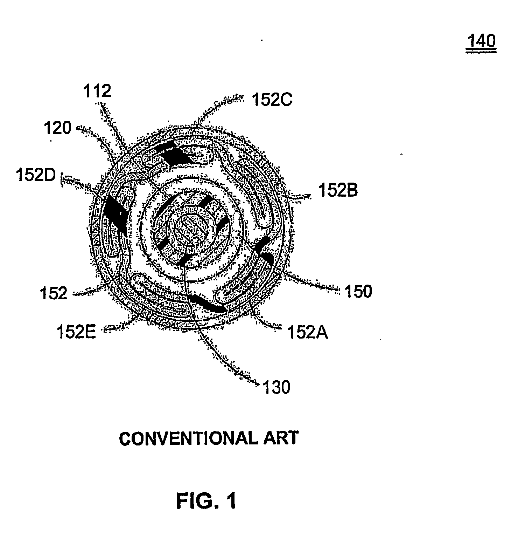

**FIG. 1** shows a cross-section of a distal portion of a conventional multifold (spiral fold) balloon stent delivery catheter **140** that includes a stent **120** and a multifold balloon angioplasty catheter **150**. Near a distal end of the multifold balloon angioplasty catheter **150,** an angioplasty balloon **152** with folds **152A, 152B, 152C, 152D** and **152E** is attached to an inner shaft (inner member) **112** which has a guide wire **130**. The inner member **112** is in fluid communication with the balloon **152**, and the inner member **112** is used to inflate and deflate the balloon **152** so as to expand the stent **120** into a wall of a vessel of a human body (not shown). A radiopaque marker band (not shown in the figures) fixed to the inner member **112** provides a reference for identifying the position of the balloon **152** and stent **120**. During expansion of the balloon **152,** the folds **152A**, **152B, 152C, 152D** and **152E** exert frictional radial forces against the inside surface of the stent **120**.

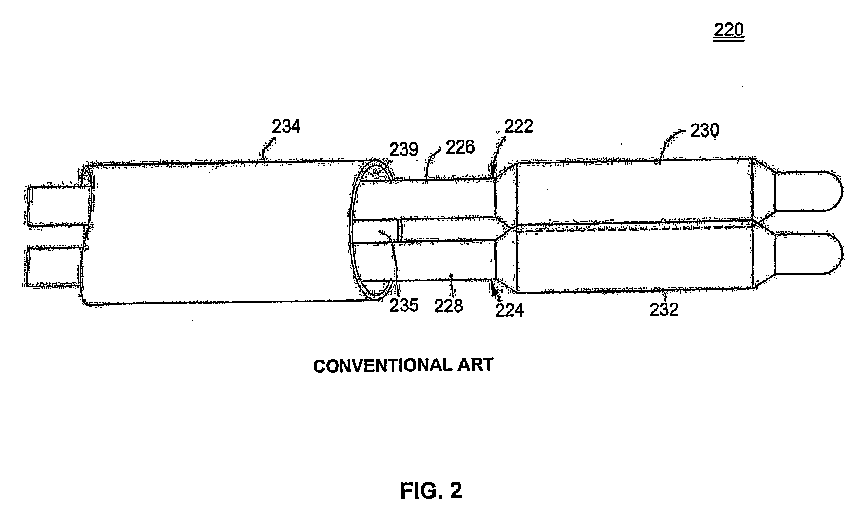

In some cases where a bifurcation in the vessel is present, a dual balloon catheter may be necessary. In **FIG. 2**, a conventional dual balloon catheter assembly **220** is shown, which includes a pair of balloon catheters **222** and **224**. Each balloon catheter includes a shaft (inner member) **226** and **228**, and balloons **230** and **232**. The balloons on each balloon catheter **226** and **228** are folded in same manner as described above with reference to **FIG. 1** such that each catheter has a circular cross-section. At least a portion of the catheters **222** and **224** may be contained within a guide catheter **234** that keeps the inner members **226** and **228** in close proximity to one another, thereby providing the assembly **220** with a relatively compact profile. The guide catheter **234** may be configured to contain the catheters **222** and **224** in the substantially circular lumen **235**.

The assembly **220** may be configured to have a circular cross-sectional profile throughout its entire length or a portion thereof. In the embodiment shown in **FIG. 2**, the guide catheter **234** is disposed about the catheters **222** and **224** and provides the assembly **220** with a substantially circular cross-section. However, due to the circular cross-section of each of the catheters **222** and **224**, a significant portion of the lumen **235** is wasted space **239**, giving the overall structure a large crossing profile. Similar conventional devices are disclosed in US 6,013,092 and US-A-2003/0083579.

Accordingly, there is a need in the art for a novel approach to folding balloons that permits reduction of the crossing profile of dual balloon catheter assemblies.

### Summary of the Invention

The present invention is directed to a balloon assembly and method as defined in the independent claims for catheter applications, that substantially obviates one or more of the problems and disadvantages of the related art.

An embodiment of the present invention includes a dual balloon assembly with a first inner member and a first balloon mounted thereon and folded in an accordion manner. A second inner member has a second balloon mounted thereon. The first balloon has a plurality of pleats on each side of the first inner member. The first balloon can have an equal number of pleats on either side of the first inner member, or can be folded in an asymmetrical manner. The first balloon can also be folded in a tri-fold accordion manner. Each of the pleats includes an ascending fold and a descending fold connected to each other by an apex. The pleats can also be connected to each other by an apex. Alternatively, the ascending and descending folds can be connected to each other by curved portions, and the pleats can also be connected to each other by curved portions.

A stent can be mounted over each of the first and second balloons. A guide catheter can be disposed over the first and second balloons. The first balloon can be formed of nylon, polyethylene teraphthalate, polyethylene, polypropylene, polyvinyl chloride, and elastomer.

The second balloon can also be folded in the accordion manner to have a plurality of pleats on either side of the second inner member. The pleats of the first and second balloons can be oriented generally perpendicular to a line connecting centers of the first and second inner members.

In another embodiment of the invention, a balloon assembly includes an inner member and a balloon mounted on the inner member and folded in an accordion manner.
The balloon includes a plurality of pleats on each side of the inner member. A stent can be mounted over the balloon, and a guide catheter can be disposed over the balloon.

In another embodiment of the invention, a balloon assembly includes a plurality of inner members and a plurality of accordion-folded balloons mounted on corresponding inner members.

In another embodiment of the invention, a method of folding a balloon includes attaching a balloon to an inner member; forming the balloon into an accordion, or batwing shape; and placing a sheath over the balloon. The forming step can include drawing a balloon through a mold that includes a plurality of ridges. The mold includes ridges that guide material of the balloon to form pleats of the accordion shape. An intravascular prosthesis, such as a stent or a stent-graft, can be placed over the balloon.
The forming step forms a plurality of pleats. The pleats can be formed symmetrically or asymmetrically about the inner member. The forming step can also form the balloon into a tri-fold accordion shape. The forming step can form a plurality of pleats of varying size.

In another embodiment of the invention, a method of forming a balloon assembly includes attaching a plurality of balloons to corresponding inner members; forming each balloon into an accordion shape; and placing a sheath over each balloon.

In another embodiment of the invention, a method of delivering a stent includes the steps of inserting a catheter into a blood vessel, the catheter including a balloon mounted on an inner member of the catheter and a stent mounted on the balloon; inflating the balloon to deliver the stent; deflating the balloon; and withdrawing the catheter from the blood vessel. The balloon mounted on the inner member is folded in an accordion manner. The stent can be a bifurcated stent, or a non-bifurcated stent.

Additional features and advantages of the invention will be set forth in the description that follows, and in part will be apparent from the description, or may be learned by practice of the invention. The advantages of the invention will be realized and attained by the structure and particularly pointed out in the written description and claims hereof as well as the appended drawings.

It is to be understood that both the foregoing general description and the following detailed description are exemplary and explanatory and are intended to provide further explanation of the invention as claimed.

### Brief Description of the Drawings

The accompanying drawings, which are included to illustrate exemplary embodiments of the invention and are incorporated in and constitute a part of this specification, illustrate embodiments of the invention and together with the description serve to explain the principles of the invention. In the drawings:

**FIG. 1** shows a partial cross-section of a conventional balloon catheter.

**FIG. 2** shows a conventional dual balloon catheter assembly.

**FIG. 3** illustrates an embodiment of a folded balloon of the present invention.

**FIG. 4** illustrates a side view of two balloons placed sequentially on an inner member and partly overlapping each other.

**FIG. 5** shows a cross-sectional view of the folded balloon of **FIG. 3**.

**FIG. 6** illustrates another embodiment of a folded balloon of the present invention, where pleats of the folded balloon have a variable size.

**FIG. 7** illustrates another embodiment of the present invention with two side-by-side folded balloons.

**FIG. 8** illustrates another embodiment of the present invention with asymmetrically folded balloons.

**FIG. 9** illustrates another embodiment of the present invention using a tri-fold accordion balloon.

**FIG. 10** illustrates a dual balloon arrangement that uses two tri-fold accordion balloons as shown in **FIG. 9****.**

**FIG. 11** illustrates yet another embodiment of the present invention that uses an arrangement of three tri-folded accordion balloons.

**FIG. 12** illustrates yet another embodiment of a folded balloon of the present invention, where for every ascending fold on one side there is a corresponding ascending fold on the other side.

**FIG. 13** illustrates yet another embodiment of a folded balloon of the present invention, with rounded portions used to connect the folds.

**FIG. 14** illustrates an isometric view and **FIG. 15** illustrates a side view of a mold used to fold a balloon of the present invention.

**FIG. 16** illustrates a cross sectional view of upper and lower portions of the mold of **FIG. 14**, and **FIG. 17** illustrates an isometric view of the lower portion of the mold of **FIG. 14**.

**FIG. 18** illustrates a cross sectional view of an embodiment of a folded balloon of the present invention with a stent mounted thereon.

### Detailed Description of Embodiments of the Invention

Reference will now be made in detail to the embodiments of the present invention, examples of which are illustrated in the accompanying drawings.

It will be appreciated that in the discussion below, the sense of direction (up, down, left, right) is purely arbitrary and is used for illustrative purposes only to help explain the drawings.

During manufacture of the balloon catheter assembly, a balloon is bonded to an inner member of a catheter. The invention is also applicable to balloons that are attached to a guide catheter, as well as any type of intravascular catheters, such as, e.g., over-the-wire catheters. A series of processing steps are then carried out on the balloon. These balloon processing steps include folding and then baking the balloon to heat set in the folded and wrapped shape. Inspection and sterilization operations are also carried out on the balloon. After this processing, the balloon is covered with a protective sleeve and shipped.

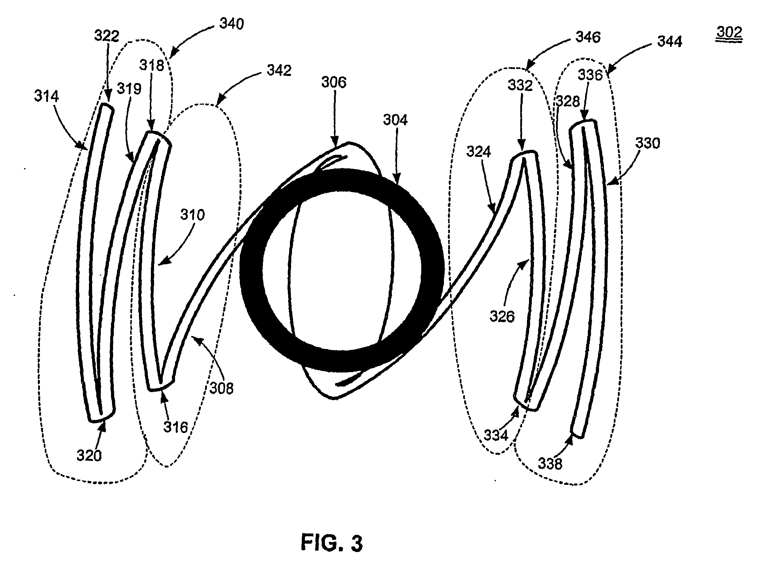

**FIG. 3** illustrates an embodiment of a folded balloon of the present invention. As shown in **FIG. 3**, a balloon assembly **302** includes an inner member **304**, which is typically 0.024 inches (0.6096 mm) in diameter and can vary between 0.020 and 0.040 inches (0.508 - 1.016 mm) in diameter. Mounted on the inner member **304** is a balloon **306** that is shown in **FIG. 3** in its folded state. The folded state shown in **FIG. 3** may be described as a batwing, or an accordion-type folding. As further shown in **FIG. 3**, the balloon **306** is folded to include a number of pleats, designated by **340**, **342**, **344** and **346** in **FIG. 3****.** Pleat **342** includes an ascending fold **308**, an apex **316**, and an ascending fold **310**. Pleat **340** is formed by a descending fold **319**, an apex **320**, an ascending fold **314**, and a termination at point **322**.

Similarly, on the right side of **FIG.3**, the balloon **306** is folded to have an ascending fold **324**, an apex **332**, a descending fold **326** (forming the pleat **346**), an apex **334**, an ascending fold **328**, an apex **336**, a descending fold **330**, and an end or termination point **338** (forming the pleat **344**).

It will be appreciated that although in **FIG. 3**, the balloon **306** is folded to have two pleats on the right, and two pleats on the left, more or fewer pleats may be used, depending on the size of the balloon **306** and the size of the inner member **304**. For example, typical balloons for coronary applications are about 2 to 5 mm in diameter, when expanded. Balloons intended for some peripheral applications may be larger, as much as 10 to 12 mm in diameter, when expanded. It will be appreciated that larger balloons will usually, though not always, use more pleats for folding the balloon **306** to form the accordion arrangement illustrated in **FIG. 3**.

Balloon **306** is usually formed of a thin pliable material capable of expanding from a compact, collapsed state to an expanded diameter. Balloon **306** may be formed from polyethylene teraphthalate (PET) using a drawing and blow molding process so as to provide biaxial orientation to the material. PET balloons exhibit the desirable properties of high burst strength and relatively low radial expansion when inflated to high pressures. Alternatively, balloon **306** may be formed from nylon, polyethylene, polypropylene, polyvinyl chloride, elastomer or other material, as would be apparent to one skilled in the relevant art. Balloon **306** is typically approximately 2-4 cm long and is attachable to a distal portion of a catheter by methods known in the art, including gluing, melting or welding.

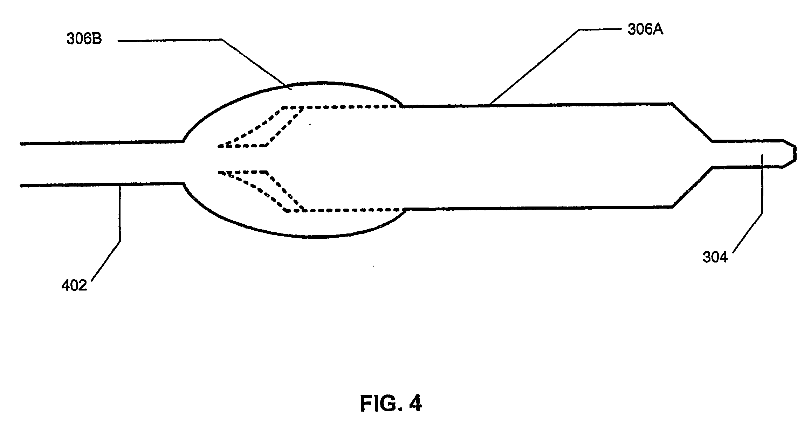

**FIG. 4** illustrates a side view of two balloons **306A, 306B** placed sequentially and partly overlapping on the inner member **304** of a catheter **402**. If such a double balloon were to be folded in a conventional manner, it would have a relatively large crossing profile. However, if folded into the accordion shapes, the crossing profile could be made relatively small.

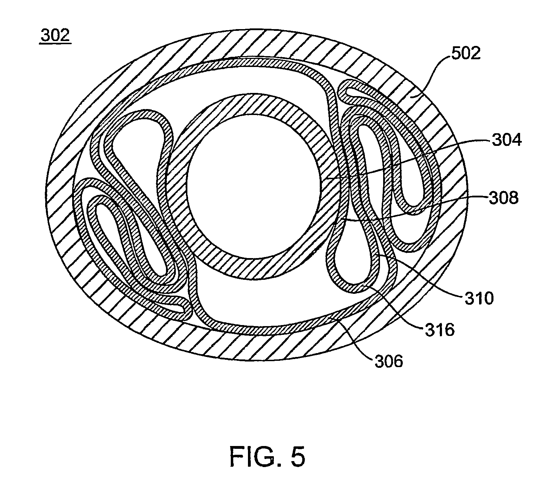

**FIG. 5** shows a photograph of a cross-sectional view of a single balloon **306** that is folded into the accordion shape, and is placed within a sheath **502** after folding.

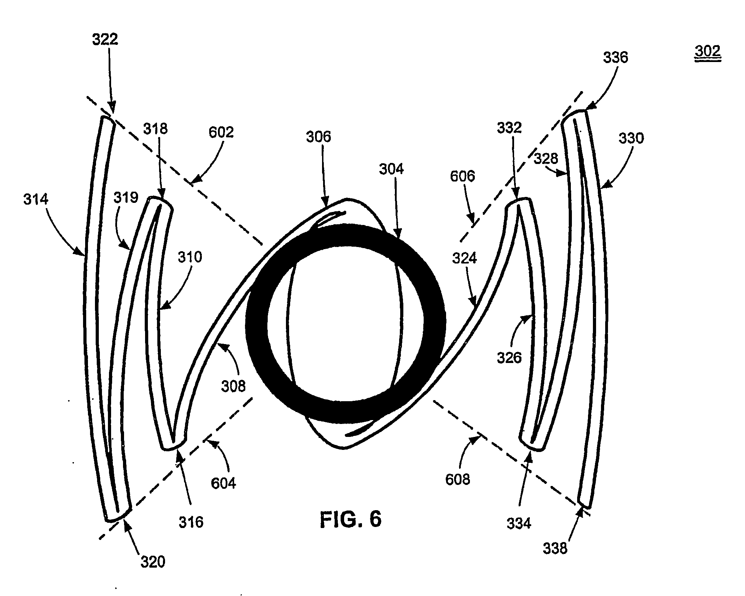

Although the embodiment shown in **FIG. 5** uses pleats are roughly comparable in dimension to the diameter of the inner member **304** (see **FIG. 3**), this need not necessarily be the case. For example, **FIG. 6** illustrates an alternative embodiment, where the pleats have a variable size, in this case, the outward pleats are larger than the inner pleats. Dashed lines **602, 604, 606** and **608** are added for illustrative purposes, to better illustrate that the outer folds are longer than the inner folds. Additionally, it is possible to have outer folds that are smaller than the inner folds (not shown in the figures). This may be advantageous when the size of the inner member **304** is relatively large. In that case, it is possible to use outer folds that are smaller than the inner folds, or folds that gradually decrease in length as one moves radially outward (left and right in **FIG. 6**) from the inner member **304**.

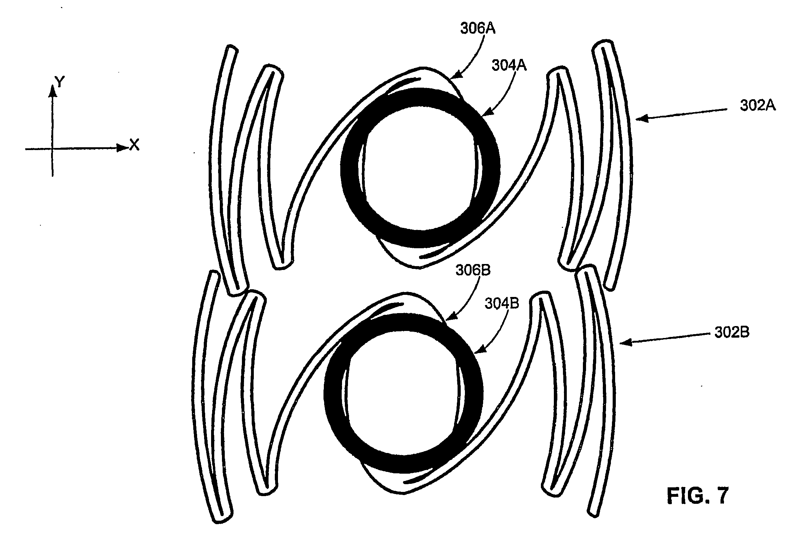

**FIG. 7** illustrates another embodiment of the present invention where two side-by-side folded balloons **302A, 302B** are used. This figure helps illustrate how the overall crossing profile of the dual balloon/catheter arrangement can be reduced. The Y dimension in **FIG.7** is substantially comparable to the dimension of the two inner members **304A, 304B** added together. When it comes to reducing the crossing profile of such a dual balloon/catheter assembly, it is typically the Y dimension that causes the primary difficulty. Note that although two distinct balloons **306A, 306B** are shown in **FIG. 7**, it is also possible to use a single (large) balloon folded into the dual accordion shape shown in **FIG. 7**. Furthermore, the folded balloon of the present invention may be used side-by-side with a conventional spiral folded balloon.

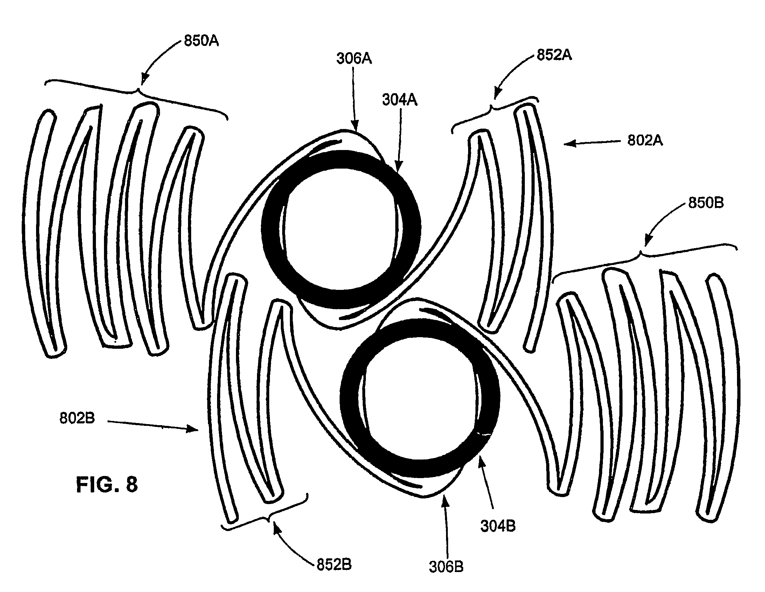

**FIG. 8** illustrates another alternative embodiment of the present invention. In the embodiments shown in, for example, **FIGS. 3****,** **6** and **7****,** the folding is symmetrical about the inner member **304**. In other words, the same number of pleats is used on both the left and the right side of the inner member **304** in the embodiments shown in **FIGS. 3****,** **6** and **7**. However, this need not be the case. As shown in **FIG. 8**, two balloons **306A, 306B** are mounted on inner members **304A, 304B**. However, the number of pleats **850A** on the left of the inner member **304A** is different from the number of pleats **852A** on the right of the inner member **304A.** Similarly, the number of pleats **850B** on the right of the inner number **304B** is different than a number of pleats **852B** on the left of the inner member **304B.** This may be particularly advantageous for acentric balloons, and may allow further reduction in the crossing profile. It will also be appreciated that the two balloon assemblies **802A** and **802B** need not be the mirror images of each other. For example, it is possible to fold one of the two balloons symmetrically, as shown, for example, in **FIG. 3**, and to fold the other balloon asymmetrically.

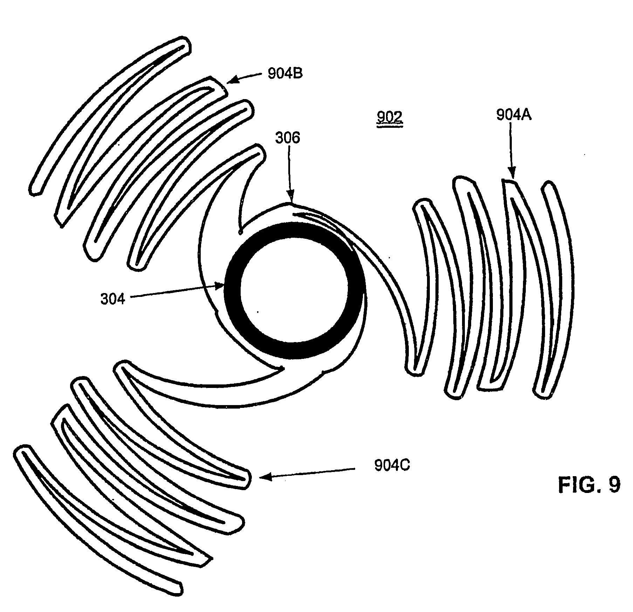

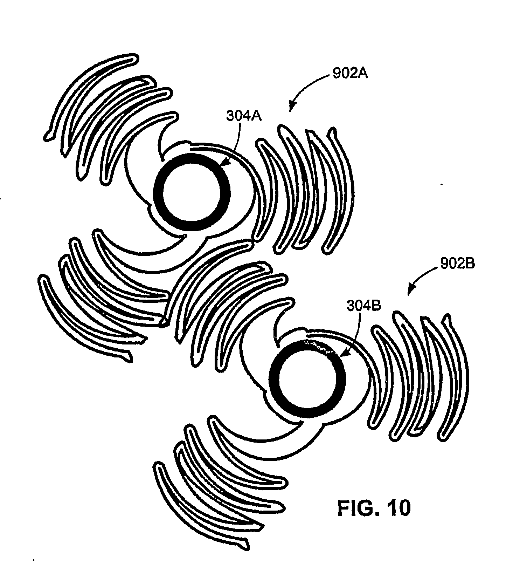

**FIG. 9** illustrates yet another alternative embodiment of the present invention. As shown in **FIG.9**, instead of a folding arrangement with two folded areas, the arrangement **902** of **FIG. 9** may be thought of as a tri-fold, or as a Y-fold. The "arms" of the Y, viz, **904A, 904B, 904C** come together at the inner member **304.** **FIG. 10** illustrates a dual balloon arrangement that uses the tri-fold illustrated in **FIG. 9**. As shown in **FIG. 10**, two tri-fold balloon assemblies **902A, 902B** are arranged side by side.

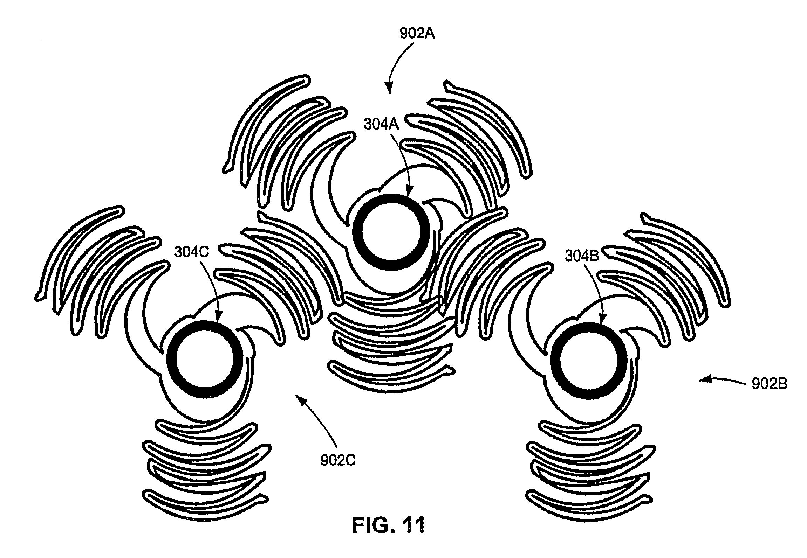

**FIG.11** illustrates yet another embodiment of the present invention that uses three tri-folded balloon assemblies **902A, 902B, 902C** arranged side-by-side. The inner members **304A, 304B, 304C** form a roughly isosceles triangle, with the tri-fold balloons **902A-C** arranged as shown in the figure, to reduce the crossing profile.

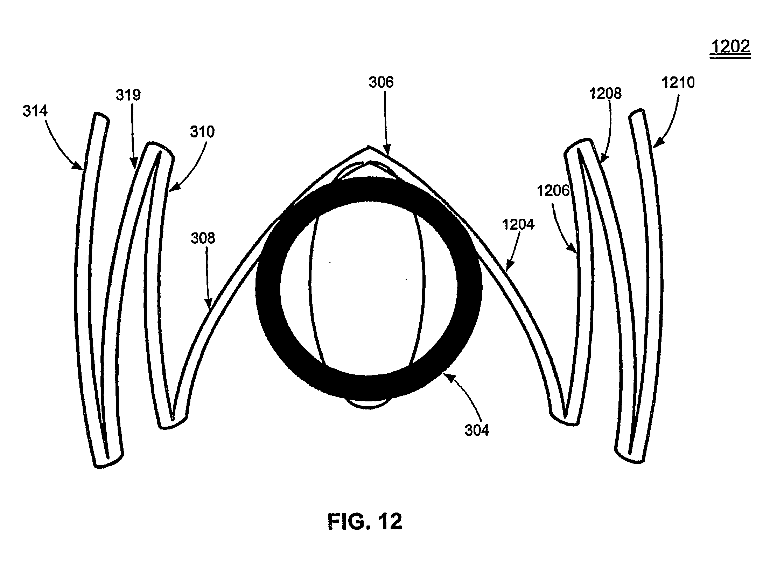

**FIG. 12** illustrates yet another alternative embodiment of the present invention. In **FIG. 3**, described above, the pleats **340, 342** on the left of the inner member **304** are mirror images of the pleats **344, 346** on the right of the inner member **304**. In other words, for every ascending fold on the left of the inner member **304**, there is a corresponding descending member on the right of the inner member **304**. In the balloon assembly **1202** shown in **FIG. 12**, for every ascending fold on the left there is a corresponding ascending fold on the right (i.e., folds **1204, 1206, 1208** and **1210**).

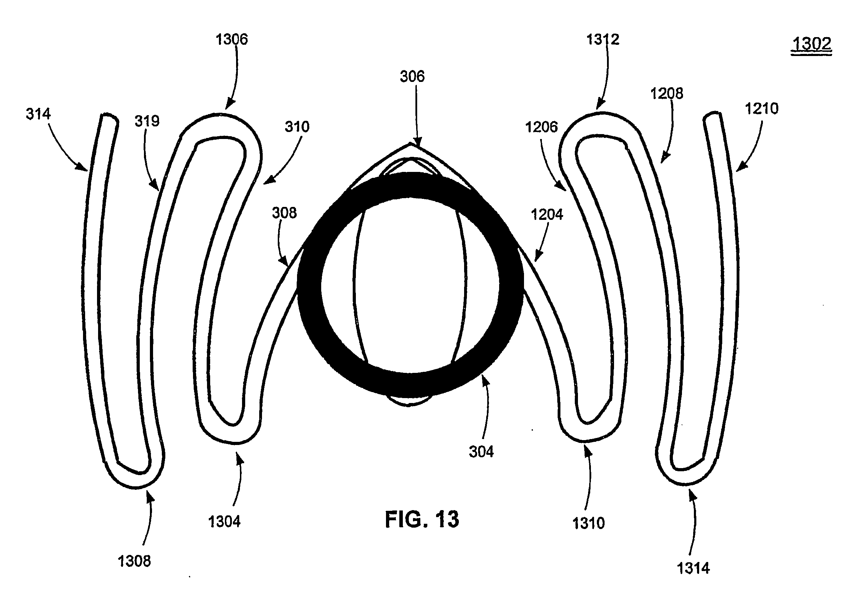

**FIG. 13** illustrates yet another alternative embodiment of the present invention. In the embodiments described above, the apices (**316, 318, 320, 332, 334, 336**) of the balloon assembly **1302** are shown as being relatively "sharp", or folded "tightly". Alternatively, as shown in **FIG. 13**, rounded portions **1304, 1306, 1308, 1310, 1312, 1314** of the balloon assembly **1302** may be used to connect the folds (see also **FIG. 5**).

In the folding stage (also called a pre-sheath stage), a balloon is folded and wrapped around a catheter. Temporary sheaths (see, e.g., **502** in **FIG. 5**) are then provided around the folded balloon **306**. One technique used to manipulate the balloon **306** in the folding stage involves use of a folding jig, or mold. First, low pressure is applied to inflate the balloon **306**. A distal end of the catheter is inserted into the mold. The balloon **306** is then pulled through the mold. The mold acts to create folds in the balloon **306**.

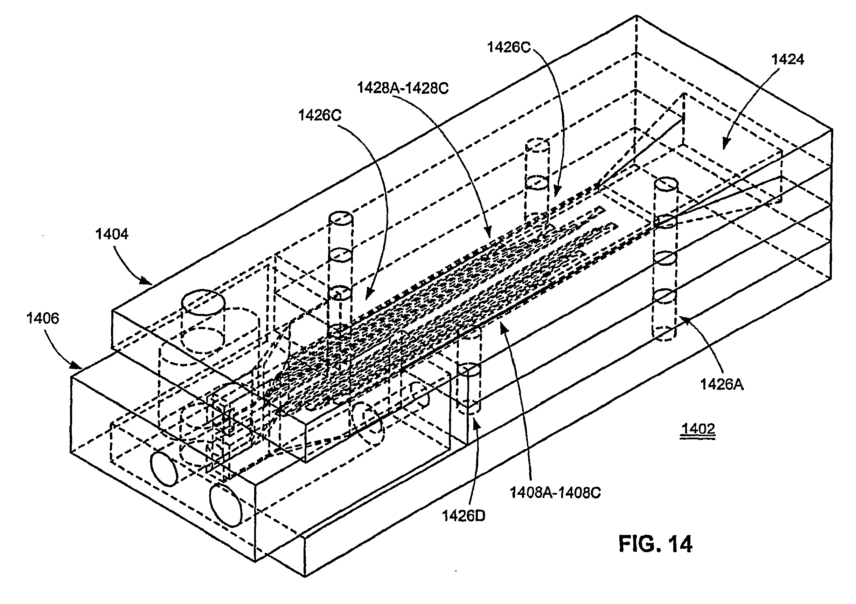

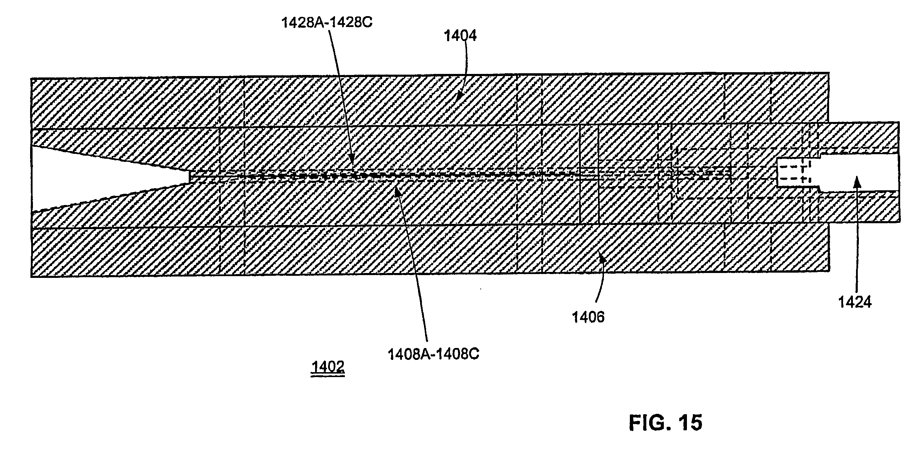

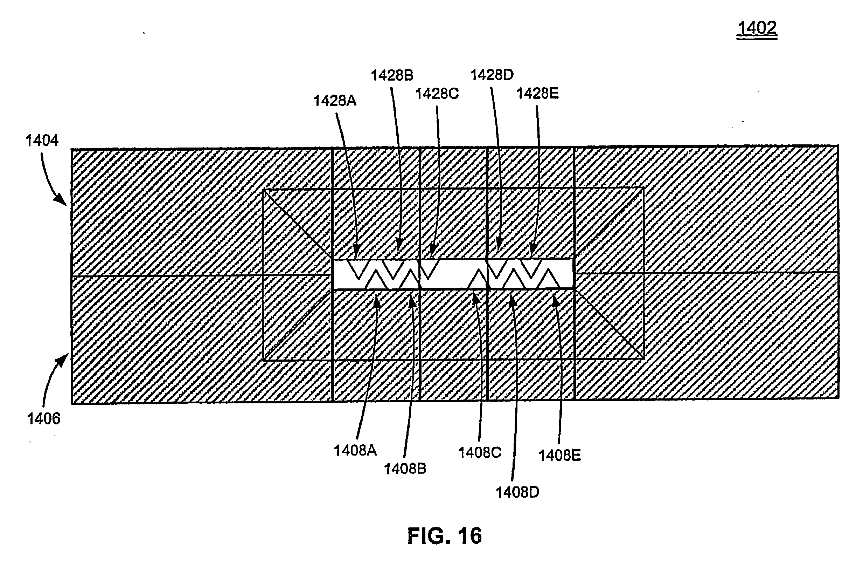

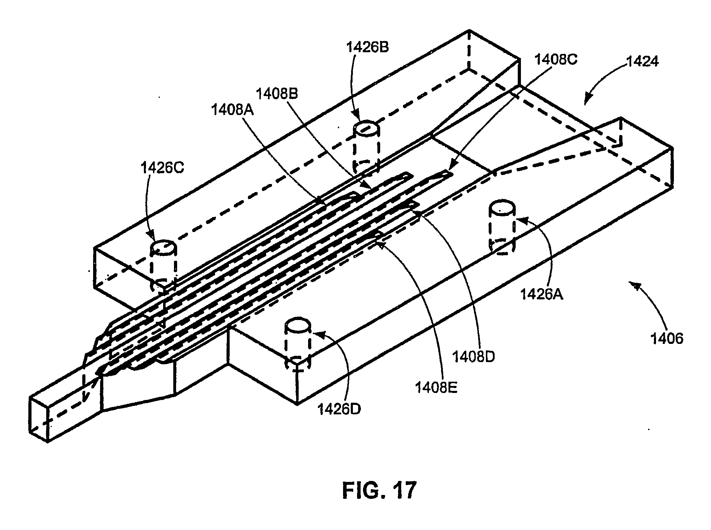

**FIG. 14** illustrates an isometric view and **FIG. 15** illustrates a side view of a mold **1402** used to fold the balloon **306** into the accordion arrangement. **FIG. 16** illustrates a cross sectional view of upper and lower portions **1404, 1406** of the mold **1402**, and **FIG. 17** illustrates an isometric view of the lower portion **1406** of the mold of **1402**. As shown in **FIGS. 14-17**, the mold **1402** includes an upper portion **1404** and a lower portion **1406.** Holes **1426A, 1426B, 1426C, 1426D** are used to align the upper and lower portions **1404, 1406**. The balloon **306** is at least partially inflated, and then drawn through the mold **1402**, thereby collapsing it and folding it.

The balloon is initially drawn from upper right towards the lower left, through an opening **1424.** Ridges **1408A-1408E** and **1428A-1428E** are used to form the accordion structure illustrated in, e.g., **FIG. 3**. As the balloon **306** is drawn further through the mold **1402**, the first folds that form are the innermost ones, since the first ridges encountered by the balloon **306** are, in this case, the longest central ridges, designated by **1408C** and **1428C**.

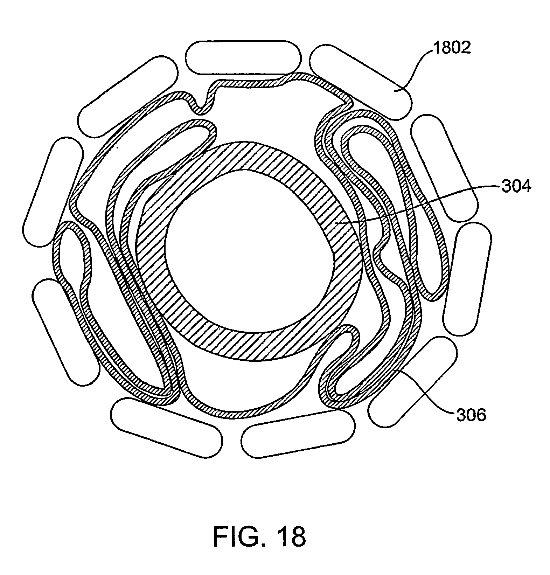

The balloon at this stage of formation, after having passed through the mold **1402**, is referred to as a "folded balloon." As the folded balloon is pulled from the mold **1402**, one or more temporary guide sheaths (see **502** in **FIG. 5**) are placed over it to enable the mounting of the stent **1802** (see **FIG. 18**) on the balloon **306**.

The temporary sheath **502** has an inner diameter (ID) that is sized to fit over the folded balloon **306**. The temporary sheath **502** is sized to fit close to the balloon **306**, but is sufficiently loose to allow room for the balloon **306** to be slightly inflated. This slight inflation or pressurization helps prevent a later-applied coating from leaking into areas between folds. The temporary sheath **502** serves as a temporary protective balloon holder.

**FIG. 18** illustrates a cross sectional view of a folded balloon **306** with the stent **1802** mounted thereon. Stent **1802** is placed over the balloon **306** and one or more additional temporary guide sheaths are placed over the entire stent and balloon package. Alternatively, two temporary sheaths (called a distal sheath and a proximal sheath) may be used. Three sheaths can be used in the case of a long balloon.

Once the folded balloon **306** and stent **1802** package is placed in a temporary sheath, a positive pressure is applied to inflate the balloon **306**. The remainder of the balloon **306** and stent **1802** located in the sheath are prevented from expanding by the sheath.

Heat is then applied to set the balloon folds. Typically, a balloon catheter is inserted into a heat oven and baked. Manual bake and set wrap machine methods can be used. Both the manual bake and set wrap machine methods heat the folded balloons at a sufficiently high temperature (approx. 170° F or 76.67°C) so that the balloon material is heat set in the folded position.

It will also be appreciated that other mold arrangements are possible and the invention is not limited to a particular arrangement of the mold **1402** shown in the figures. Also, for the tri-fold accordion (see **FIG. 9**), a three-piece mold (with ridges, similar to what is shown in **FIGS. 14-17**) may be used, with the surfaces arranged at, e.g., about 120 degrees, and pieces of the mold coming together in a manner to form the tri-fold, such as shown in **FIG. 9**.

The invention has a number of advantages. For example, some of the embodiments allow reduction the crossing profile, particularly in the case of a dual balloon arrangement commonly used in implantation of a bifurcated stent structure. Expansion of the stent is more uniform, since, particularly for the tri-fold arrangement, the direction of the expanding force of the balloon is radially outward (compared to the partly tangential direction in a conventional spiral-folded balloon). Similarly, the arrangement illustrated in some of the figures allows reduction of the crossing profile of a side-by-side balloon system. Additionally, the described embodiments allow expansion of the balloon, and corresponding expansion of the stent, without the balloon scraping against the stent. This is particularly important if the stent has a pharmacological coating on it.

Additionally, it may be desirable to have different bending characteristics in the X and Y direction (see **FIG. 7****,** sometimes referred to as "preferential bending"), depending on the patient anatomy. The present invention allows a preferred direction of bending for each balloon, which may be used to counteract the preferred direction of bending of a conventional two-balloon structure. In **FIG. 2**, it is generally easier to bend the entire structure, which consists of two inner members and two balloons, in the "out of the paper" direction as opposed to the "vertical" direction in the figure. The arrangement as shown in **FIG. 7** may be used to counteract the preferential bending.

Additionally, because a structure with a roughly "rectangular" cross section has a longer circumference than a structure with a circular cross section (such as in a conventional spiral-folded balloon), it is possible to "fit more stent material" around the balloon, for the same crossing profile. Also, it is believed that stent retention is improved, compared to conventional folding arrangements.

It will be understood by those skilled in the art that various changes in form and details may be made therein without departing from the scope of the invention as defined in the appended claims.

## Claims

1. A balloon assembly for use in the medical field comprising:
an inner member (304); and
a balloon (306) mounted on the inner member; **characterised in that** the balloon is folded in an accordion manner.

2. The balloon assembly of claim 1, wherein the balloon comprises a plurality of pleats (340, 342, 344, 346) on either side of the inner member.

3. The balloon assembly of claim 2, wherein the balloon comprises an equal number of pleats on either side of the inner member.

4. The balloon assembly of claim 2, wherein the balloon is folded in an asymmetrical manner.

5. The balloon assembly of claim 1, wherein the balloon is folded in a tri-fold accordion manner.

6. The balloon assembly of claim 1, further comprising a stent (1802) mounted over the balloon.

7. The balloon assembly of claim 6, further comprising a catheter connected to the balloon.

8. The balloon assembly of claim 1, wherein the balloon is formed of any of polyethylene teraphthalate, polyethylene, polypropylene, polyvinyl chloride, and elastomer.

9. The balloon assembly of claim 1, wherein the balloon is folded into a plurality of pleats that are mirror images of each other about the inner member.

10. The balloon assembly of claim 1, wherein the balloon is folded into a plurality of pleats, each of the pleats comprising an ascending fold and a descending fold, the ascending and descending folds connected by an apex.

11. The balloon assembly of claim 10, wherein pleats are connected by an apex.

12. The balloon assembly of claim 1, wherein the balloon is folded into a plurality of pleats, each of the pleats comprising an ascending fold and a descending fold, the ascending and descending folds connected by a curved portion.

13. The balloon assembly of claim 12, wherein pleats are connected by a curved portion.

14. A dual balloon assembly comprising:
a balloon assembly as defined in any preceding manner; and further comprising
a second inner member (304B); and
a second balloon (302B) mounted on the second inner member.

15. The dual balloon assembly of claim 14, further comprising a bifurcated stent mounted over the balloons.

16. The dual balloon assembly of claim 15, further comprising a catheter connected to the balloons.

17. The dual balloon assembly of claim 14, wherein the inner member (304A) is hollow.

18. The dual balloon assembly of claim 14, wherein the second balloon is folded in the accordion manner and comprises a plurality of pleats on either side of the second inner member.

19. The dual balloon assembly of claim 18, wherein the balloons, in their folded state, are oriented generally perpendicular to a line connecting centers of the first and second inner members.

20. The dual balloon assembly of claim 14, wherein the second balloon is folded in the accordion manner and comprises an equal number of pleats on either side of the second inner member.

21. The dual balloon assembly of claim 14, wherein the second balloon is folded in an asymmetrical manner.

22. The dual balloon assembly of claim 14, wherein the second balloon is folded in a tri-fold accordion manner.

23. The dual balloon assembly of claim 14, wherein the second balloon is folded into a plurality of pleats, each of the pleats comprising an ascending fold and a descending fold, the ascending and descending folds connected by an apex.

24. The dual balloon assembly of claim 23, wherein adjacent pleats of the second balloon are connected by an apex.

25. The dual balloon assembly of claim 14, wherein second balloon is folded into a plurality of pleats, each of the pleats comprising an ascending fold and a descending fold, the ascending and descending folds connected by a curved portion.

26. The dual balloon assembly of claim 25, wherein the second balloon is folded into a plurality of pleats, adjacent pleats being connected by a curved portion.

27. A balloon assembly as claimed in any of claims 1 to 5, 8 or 9, comprising:
a plurality of said inner members (304); and
a plurality of said balloons (302) mounted on corresponding inner members.

28. The balloon assembly of claim 27, further comprising an implantable prosthesis mounted over the accordion-folded balloons.

29. The balloon assembly of claim 28, further comprising a catheter connected to the accordion-folded balloons.

30. A method of folding a balloon for use in the medical field comprising:
attaching a balloon (302) to an inner member (304);
forming the balloon into an accordion shape; and
placing a sheath (502) over the balloon.

31. The method of claim 30, wherein the forming step includes drawing a balloon through a mold (1402) that includes a plurality of ridges (1408A - 1408E; 1428A - 1428E).

32. The method of claim 31, wherein the ridges guide material of the balloon to form pleats of the accordion shape.

33. The method of claim 31, further comprising the step of placing an intravascular prosthesis over the balloon.

34. The method of claim 31, wherein the forming step forms a plurality of pleats.

35. The method of claim 34, wherein the pleats are formed symmetrically about the inner member.

36. The method of claim 34, wherein the pleats are formed asymmetrically about the inner member.

37. The method of claim 30, wherein the forming step forms a tri-fold shape.

38. The method of claim 30, wherein the forming step forms a plurality of pleats of varying size.

39. The method of any of claims 30 to 3 8, comprising:
attaching a plurality of said balloons to corresponding inner members;
forming each balloon into an accordion shape; and
placing a sheath over each balloon.

## Patentansprüche

1. Ballonanordnung für die Verwendung im medizinischen Bereich, mit:
einem inneren Element (304); und
einem Ballon (306), der an dem inneren Element angebracht ist, **dadurch gekennzeichnet, dass** der Ballon akkordeonartig gefaltet ist.

2. Ballonanordnung nach Anspruch 1, wobei der Ballon mehrere Falten (340, 342, 344, 346) beiderseits des inneren Elements aufweist.

3. Ballonanordnung nach Anspruch 2, wobei der Ballon auf jeder Seite des inneren Elements die gleiche Anzahl von Falten aufweist.

4. Ballonanordnung nach Anspruch 2, wobei der Ballon auf asymmetrische Weise gefaltet ist.

5. Ballonanordnung nach Anspruch 1, wobei der Ballon dreifach akkordeonartig gefaltet ist.

6. Ballonanordnung nach Anspruch 1, die ferner einen über dem Ballon angebrachten Stent (1802) aufweist.

7. Ballonanordnung nach Anspruch 6, die ferner einen mit dem Ballon verbundenen Katheter aufweist.

8. Ballonanordnung nach Anspruch 1, wobei der Ballon aus irgend einem der folgenden Materialien gebildet ist: Polyethylen-Terephthalat, Polyethylen, Polypropylen, Polyvinylchlorid und Elastomer.

9. Ballonanordnung nach Anspruch 1, wobei der Ballon in mehrere Falten gefaltet ist, die um das innere Element Spiegelbilder voneinander sind.

10. Ballonanordnung nach Anspruch 1, wobei der Ballon in mehrere Falten gefaltet ist, wobei jede der Falten eine ansteigende Falte und eine absteigende Falte enthält, wobei die ansteigenden Falten und die absteigenden Falten durch einen Scheitel miteinander verbunden sind.

11. Ballonanordnung nach Anspruch 10, wobei Falten durch einen Scheitel verbunden sind.

12. Ballonanordnung nach Anspruch 1, wobei der Ballon in mehrere Falten gefaltet ist, wobei jede der Falten eine ansteigende Falte und eine absteigende Falte enthält, wobei die ansteigenden Falten und die absteigenden Falten durch einen gekrümmten Abschnitt miteinander verbunden sind.

13. Ballonanordnung nach Anspruch 12, wobei die Falten durch einen gekrümmten Abschnitt miteinander verbunden sind.

14. Doppelballonanordnung, mit:
einer Ballonanordnung gemäß einer der vorstehenden Definitionen, und ferner mit
einem zweiten inneren Element (304B); und
einem zweiten Ballon (302B), der an dem zweiten inneren Element angebracht ist.

15. Doppelballonanordnung nach Anspruch 14, ferner mit einem gabelförmigen Stent, der über den Ballonen angebracht ist.

16. Doppelballonanordnung nach Anspruch 15, ferner mit einem Katheter, der mit den Ballonen verbunden ist.

17. Doppelballonanordnung nach Anspruch 14, wobei das innere Element (304A) hohl ausgebildet ist.

18. Doppelballonanordnung nach Anspruch 14, wobei der zweite Ballon akkordeonartig gefaltet ist und beiderseits des zweiten inneren Elements mehrere Falten aufweist.

19. Doppelballonanordnung nach Anspruch 18, wobei die Ballone in ihrem gefalteten Zustand im Allgemeinen senkrecht zu einer Linie orientiert sind, die die Zentren des ersten und der zweiten inneren Elemente miteinander verbindet.

20. Doppelballonanordnung nach Anspruch 14, wobei der zweite Ballon akkordeonartig gefaltet ist und die gleiche Anzahl von Falten beiderseits des zweiten inneren Elements aufweist.

21. Doppelballonanordnung nach Anspruch 14, wobei der zweite Ballon asymmetrisch gefaltet ist.

22. Doppelballonanordnung nach Anspruch 14, wobei der zweite Ballon dreifach akkordeonartig gefaltet ist.

23. Doppelballonanordnung nach Anspruch 14, wobei der zweite Ballon in mehrere Falten gefaltet ist, wobei jede der Falten eine ansteigende Falte und eine absteigende Falte enthält, wobei die ansteigenden Falten und die absteigenden Falten durch einen Scheitel miteinander verbunden sind.

24. Doppelballonanordnung nach Anspruch 23, wobei benachbarte Falten des zweiten Ballons durch einen Scheitel miteinander verbunden sind.

25. Doppelballonanordnung nach Anspruch 14, wobei der zweite Ballon in mehrere Falten gefaltet ist, wobei jede der Falten eine ansteigende Falte und eine absteigende Falte aufweist, wobei die ansteigenden Falten und die absteigenden Falten durch einen gekrümmten Abschnitt miteinander verbunden sind.

26. Doppelballonanordnung nach Anspruch 25, wobei der zweite Ballon in mehrere Falten gefaltet ist, wobei benachbarte Falten durch einen gekrümmten Abschnitt miteinander verbunden sind.

27. Ballonanordnung nach einem der Ansprüche 1 bis 5, 8 oder 9, mit:
mehreren der inneren Elemente (304); und
mehreren der Ballone (302), die an entsprechenden inneren Elementen angebracht sind.

28. Ballonanordnung nach Anspruch 27, ferner mit einer implantierbaren Prothese, die über den akkordeonartig gefalteten Ballonen angebracht ist.

29. Ballonanordnung nach Anspruch 28, ferner mit einem Katheter, der mit den akkordeonartig gefalteten Ballonen verbunden ist.

30. Verfahren zum Falten eines Ballons zur Verwendung im medizinischen Bereich, das umfasst:
Befestigen eines Ballons (302) an einem inneren Element (304);
Formen des Ballons in eine Akkordeonform; und
Anordnen einer Hülle (502) über dem Ballon.

31. Verfahren nach Anspruch 30, wobei der Schritt des Formens das Ziehen eines Ballons durch eine Form (1402), die mehrere Rippen (1408A-1408E; 1428A-1428D) enthält, umfasst.

32. Verfahren nach Anspruch 31, wobei die Rippen Material des Ballons führen, um Falten der Akkordeonform zu bilden.

33. Verfahren nach Anspruch 31, das ferner den Schritt des Anordnens einer intravaskulären Prothese über dem Ballon umfasst.

34. Verfahren nach Anspruch 31, wobei der Formungsschritt mehrere Falten formt.

35. Verfahren nach Anspruch 34, wobei die Falten symmetrisch um das innere Organ geformt werden.

36. Verfahren nach Anspruch 34, wobei die Falten asymmetrisch um das innere Organ geformt werden.

37. Verfahren nach Anspruch 30, wobei der Schritt des Formens eine dreifach gefaltete Gestalt bzw. Form formt.

38. Verfahren nach Anspruch 30, wobei der Schritt des Formens mehrere Falten mit unterschiedlicher Größe formt.

39. Verfahren nach einem der Ansprüche 30 bis 38, das umfasst:
Befestigen mehrerer der Ballone an entsprechenden inneren Elementen;
Formen jedes Ballons in eine akkordeonartige Gestalt; und
Anordnen einer Hülle über jedem Ballon.

## Revendications

1. Ensemble à ballon destiné à être utilisé dans le domaine médical, comportant :
un élément intérieur (304) ; et
un ballon (306) monté sur l'élément intérieur, **caractérisé en ce que** le ballon est plié d'une manière en accordéon.

2. Ensemble à ballon selon la revendication 1, dans lequel le ballon comporte une pluralité de plis (340, 342, 344, 346) de chaque côté de l'élément intérieur.

3. Ensemble à ballon selon la revendication 2, dans lequel le ballon comporte un nombre égal de plis de chaque côté de l'élément intérieur.

4. Ensemble à ballon selon la revendication 2, dans lequel le ballon est plié d'une manière asymétrique.

5. Ensemble à ballon selon la revendication 1, dans lequel le ballon est plié d'une manière en accordéon à trois plis.

6. Ensemble à ballon selon la revendication 1, comportant en outre une endoprothèse vasculaire (1802) montée au-dessus du ballon.

7. Ensemble à ballon selon la revendication 6, comportant en outre un cathéter relié au ballon.

8. Ensemble à ballon selon la revendication 1, dans lequel le ballon est formé en un matériau quelconque parmi du polyéthylènetéréphtalate, du polyéthylène, du polypropylène, du polychlorure de vinyle, et un élastomère.

9. Ensemble à ballon selon la revendication 1, dans lequel le ballon est plié en une pluralité de plis qui sont des images miroirs les uns des autres autour de l'élément intérieur.

10. Ensemble à ballon selon la revendication 1, dans lequel le ballon est plié en une pluralité de plis, chacun des plis comportant un pli ascendant et un pli descendant, les plis ascendant et descendant étant reliés par un sommet.

11. Ensemble à ballon selon la revendication 10, dans lequel les plis sont reliés par un sommet.

12. Ensemble à ballon selon la revendication 1, dans lequel le ballon est plié en une pluralité de plis, chacun des plis comportant un pli montant et un pli descendant, les plis montant et descendant étant reliés par une partie incurvée.

13. Ensemble à ballon selon la revendication 12, dans lequel les plis sont reliés par une partie incurvée.

14. Ensemble à deux ballons, comportant :
un ensemble à ballon tel que défini selon une quelconque manière précédente ; et comportant en outre
un second élément intérieur (304B) ; et
un second ballon (302B) monté sur le second élément intérieur.

15. Ensemble à deux ballons selon la revendication 14, comportant en outre une endoprothèse vasculaire bifurquée montée au-dessus des ballons.

16. Ensemble à deux ballons selon la revendication 15, comportant en outre un cathéter relié aux ballons.

17. Ensemble à deux ballons selon la revendication 14, dans lequel l'élément intérieur (304A) est creux.

18. Ensemble à deux ballons selon la revendication 14, dans lequel le second ballon est plié d'une manière en accordéon, et comporte une pluralité de plis de chaque côté du second élément intérieur.

19. Ensemble à deux ballons selon la revendication 18, dans lequel les ballons, dans leur état plié, sont orientés généralement perpendiculaires à une ligne reliant les centres des premier et second éléments intérieurs.

20. Ensemble à deux ballons selon la revendication 14, dans lequel le second ballon est plié d'une manière en accordéon, et comporte un nombre égal de plis de chaque côté du second élément intérieur.

21. Ensemble à deux ballons selon la revendication 14, dans lequel le second ballon est plié d'une manière asymétrique.

22. Ensemble à deux ballons selon la revendication 14, dans lequel le second ballon est plié d'une manière en accordéon à trois plis.

23. Ensemble à deux ballons selon la revendication 14, dans lequel le second ballon est plié en une pluralité de plis, chacun des plis comportant un pli montant et un pli descendant, les plis montant et descendant étant reliés par un sommet.

24. Ensemble à deux ballons selon la revendication 23, dans lequel des plis adjacents du second ballon sont reliés par un sommet.

25. Ensemble à deux ballons selon la revendication 14, dans lequel le second ballon est plié en une pluralité de plis, chacun des plis comportant un pli montant et un pli descendant, les plis montant et descendant étant reliés par une partie incurvée.

26. Ensemble à deux ballons selon la revendication 25, dans lequel le second ballon est plié en une pluralité de plis, des plis adjacents étant reliés par une partie incurvée.

27. Ensemble à ballons selon l'une quelconque des revendications 1 à 5, 8 ou 9, comportant :
une pluralité desdits éléments intérieurs (304) ; et
une pluralité desdits ballons (302) montés sur des éléments intérieurs correspondants.

28. Ensemble à ballons selon la revendication 27, comportant en outre une prothèse implantable montée sur lesdits ballons pliés en accordéon.

29. Ensemble à ballons selon la revendication 28, comportant en outre un cathéter relié aux ballons pliés en accordéon.

30. Procédé pour plier un ballon destiné être utilisé dans le domaine médical, comportant les étapes consistant à :
fixer un ballon (302) sur un élément intérieur (304) ;
former le ballon en une forme d'accordéon ; et
placer une gaine (502) au-dessus du ballon.

31. Procédé selon la revendication 30, dans lequel l'étape de formation comporte l'étirage d'un ballon à travers un moule (1402) qui comporte une pluralité de stries (1408A à 1408E ; 1428A à 1428E).

32. Procédé selon la revendication 31, dans lequel les stries guident le matériau du ballon pour former des plis en forme d'accordéon.

33. Procédé selon la revendication 31, comportant en outre l'étape consistant à placer une prothèse intravasculaire au-dessus du ballon.

34. Procédé selon la revendication 31, dans lequel l'étape de formation forme une pluralité de plis.

35. Procédé selon la revendication 34, dans lequel les plis sont formés de manière symétrique autour de l'élément intérieur.

36. Procédé selon la revendication 34, dans lequel les plis sont formés de manière asymétrique autour de l'élément intérieur.

37. Procédé selon la revendication 30, dans lequel l'étape de formation forme une forme à trois plis.

38. Procédé selon la revendication 30, dans lequel l'étape de formation forme une pluralité de plis d'une dimension variable.

39. Procédé selon l'une quelconque des revendications 30 à 38, comportant les étapes consistant à :
fixer une pluralité desdits ballons sur des éléments intérieurs correspondants ;
former chaque ballon en une forme d'accordéon ; et
placer une gaine au-dessus de chaque ballon.
